(19) Europäisches Patentamt

European Patent Office

Office européen des brevets

(11) Veröffentlichungsnummer: **0 554 292 B1**

(12) **EUROPÄISCHE PATENTSCHRIFT**

(45) Veröffentlichungstag der Patentschrift: **21.12.94**

(21) Anmeldenummer: **91918146.1**

(22) Anmeldetag: **16.10.91**

(86) Internationale Anmeldenummer:
**PCT/EP91/01966**

(87) Internationale Veröffentlichungsnummer:
**WO 92/07543 (14.05.92 92/11)**

(51) Int. Cl.⁵: **A61K  7/00**, A61K 7/06,
A61K 7/48

(54) **ÖL-IN-WASSER-EMULSIONEN.**

(30) Priorität: **25.10.90 DE 4033928**

(43) Veröffentlichungstag der Anmeldung:
**11.08.93 Patentblatt  93/32**

(45) Bekanntmachung des Hinweises auf die
Patenterteilung:
**21.12.94 Patentblatt  94/51**

(84) Benannte Vertragsstaaten:
**BE DE ES FR GB IT NL**

(56) Entgegenhaltungen:
**EP-A- 0 428 157**

**World patents index latest, week 8938, Derwent Publications Ltd, London, GB ; AN 276086**

**World patents index latest, week 8608, Derwent Publications Ltd, London, GB, AN 86-051904**

(73) Patentinhaber: **Henkel Kommanditgesellschaft auf Aktien**

**D-40191 Düsseldorf (DE)**

(72) Erfinder: **KAWA, Rolf**
**Fontanestrasse 28**
**D-4019 Monheim 2 (DE)**
Erfinder: **ANSMANN, Achim**
**Kirchberg 25**
**D-4006 Erkrath (DE)**
Erfinder: **WEUTHEN, Manfred**
**Hermann-Löns-Weg 102**
**D-5650 Soligen 11 (DE)**
Erfinder: **TESMANN, Holger**
**Vennstrasse 61**
**D-4000 Düsseldorf 12 (DE)**
Erfinder: **FÖRSTER, Thomas**
**Adalbert-Stifter-Strasse 15**
**D-4006 Erkrath (DE)**

Chemical abstracts, vol. 84, n 7, Columbus, Ohio, US ; Niiya, I et al "Effects of emulsifiers on the crystal growth of edible solid fats. IV. effects of propylene glycol esters of fatty acids and unsaturated fatty acid monoglycerides"

**Beschreibung**

Die Erfindung betrifft lagerstabile Öl-in Wasser-Emulsionen, fortan als O/W-Emulsionen bezeichnet, mit einem Gehalt an Alkylglucosiden und einem Partialglycerid mit einem Monoglyceridanteil von 60 bis 95 Gew.-% sowie Emulgatorkonzentrate enthaltend Alkylglucoside, ein Partialglycerid mit einem Monoglyceridanteil von 60 bis 95 Gew.-% und einen langkettigen Alkohol.

Die Verwendung von Alkylglucosiden auf dem Gebiet der Kosmetik ist literaturbekannt. So empfiehlt die Firma Rohm & Haas in ihrem Merkblatt TRITON CG-110 die Verwendung eines Alkylglucosids auf Basis Octanol/Decanol (50 : 50) als Hilfsemulgator für kosmetische Emulsionen, z.B. für Hautcremes und Lotionen. Die japanische Patentanmeldung JP 89/203 036 (Shiseido) beschreibt kosmetische Emulsionen, die ein $C_{8-24}$-glucosid gemeinsam mit einem Polyol mit wenigstens drei Hydroxylgruppen, z.B. Glycerin, und einer Ölkomponente enthalten. Schließlich beschreiben Yoshitomi Pharm. Ind. in der JP 86/5005 die Verwendung von Alkyl($C_{8-18}$)-glucosiden als Befeuchtungsmittel, z.B. in kosmetischen Cremes.

Alkylglucoside eignen sich als Emulgatoren zur Herstellung von O/W-Emulsionen. Es wurde beobachtet, daß die in derartigen Emulsionen enthaltenen Alkylglucoside eine ausgeprägte Kristallisationsneigung besitzen, die sich auch durch nichtionische, anionische oder kationische Coemulgatoren nicht unterdrücken läßt. Das Kristallwachstum macht sich makroskopisch durch eine Strukturverschlechterung bemerkbar. Derartige Kristallstrukturen zerstören die homogene Tröpfchenverteilung mit der Folge einer unerwünschten Destabilisierung und schließlich Brechung der Emulsionen. Die Aufgabe der vorliegenden Erfindung bestand deshalb darin, lagerstabile O/W-Emulsionen mit einem Gehalt an Alkylglucosiden zugänglich zu machen.

Diese Aufgabe wurde erfindungsgemäß gelöst durch Öl-in-Wasser-Emulsionen mit einem Gehalt an Alkylglucosiden, dadurch gekennzeichnet, daß sie enthalten

(A) einen oder mehrere wasserunlösliche Ölkörper,

(B) als Emulgator ein oder mehrere Alkylglucoside der allgemeinen Formel RO(G)$_x$, worin bedeuten

- R einen linearen, gesättigten Alkylrest mit 8 bis 22 C-Atomen
- G eine Glucoseeinheit
- x eine beliebige Zahl zwischen 1 und 10,

dadurch gekennzeichnet, daß sie

(C) als Kristallisationsinhibitor ein Fettsäurepartialglycerid mit einem Monoanteil von 60 bis 95 Gew.-%.

Eine bevorzugte Ausführungsform der vorliegenden Erfindung ist dadurch gekennzeichnet, daß die erfindungsgemäßen Emulsionen den wasserunslöslichen Ölkörper (A) in einer Menge von 5 bis 30 Gew.-%, das Alkylglucosid (B) in einer Menge von 2 bis 15 Gew.-% und das Fettsäurepartialglycerid (C) in einer Menge von 2 bis 20 Gew.-% enthält.

Bei den erfindungsgemäßen O/W-Emulsionen sind selbst bei 400-facher Vergrößerung im Lichtmikroskop keine Kristalle von Alkylglucosiden mehr zu erkennen. Diese Emulsionen sind homogen und lagerstabil.

Als Ölkörper (A) können an sich alle bei Raumtemperatur (20 °C) flüssigen, wasserunlöslichen, verzweigten oder linearen, physiologisch verträglichen aliphatischen Kohlenwasserstoffe, Ether oder Ester eingesetzt werden. Es können aber auch feste oder höherschmelzende Paraffine, Ester, Wachse oder Fette mitverwendet werden.

Als Ölkörper gut geeignet sind aus der Gruppe der aliphatischen Kohlenwasserstoffe z.B. Squalan, Squalene, Dioctylcyclohexan, Paraffinum perliquidum und paraffinum subliquidum und Isohexadecan (hydriertes Polybutylen).

Als Ölkörper gut geeignet sind ferner Ester von drei- und mehrwertigen Alkoholen, insbesondere pflanzliche Triglyceride, z.B. Olivenöl, Mandelöl, Erdnußöl, Sonnenblumenöl oder auch die Ester des Pentaerythrits mit z.B. Pelargonsäure oder Ölsäure.

Als Ölkörper gut geeignet sind weiterhin Mono- und Diester der allgemeinen Formeln I, II und III

(I)     $R^1$-COOR$^2$

(II)     $R^2$-OOC-$R^3$-COOR$^2$

(III)     $R^1$-COO-$R^3$-OOC-$R^1$

worin $R^1$ eine Alkylgruppe mit 8 bis 22 C-Atomen und $R^2$ eine Alkylgruppe mit 3 bis 22 C-Atomen und $R^3$ Alkylengruppen mit 2 bis 16 C-Atomen bedeuten und die mindestens 11 und höchstens 40 C-Atome enthalten.

Ölkörper vom Typ der Mono- und Diester der Formeln (I), (II) und (III) sind als kosmetische und pharmazeutische Ölkomponenten sowie als Gleit- und Schmiermittelkomponenten bekannt. Unter den Mono- und Diestern dieser Art kommt den bei Raumtemperatur (20 °C) flüssigen Produkten die größte Bedeutung zu. Als Ölkörper geeignete Monoester (I) sind z.B. die Isopropylester von Fettsäuren mit 12 - 22 C-Atomen, wie z.B. Isopropylmyristat, Isopropylpalmitat, Isopropylstearat, Isopropyloleat. Andere geeignete Monoester sind z.B. n-Butylstearat, n-Hexyllaurat, n-Decyloleat, Isooctylstearat, Isononylpalmitat, Isononyl-isononanoat, 2-Ethylhexyl-palmitat, 2-Ethylhexyl-laurat, 2-Hexyldecyl-stearat, 2-Octyldodecyl-palmitat, Oleyloleat, Oleylerucat, Erucyloleat sowie Ester, die aus technischen aliphatischen Alkoholgemischen und technischen aliphatischen Carbonsäuren erhältlich sind, z.B. Ester aus gesättigten und ungesättigten Fettalkoholen mit 12 - 22 C-Atomen und gesättigten und ungesättigten Fettsäuren mit 12 - 22 C-Atomen, wie sie aus tierischen und pflanzlichen Fetten zugänglich sind. Geeignet sind auch natürlich vorkommende Monoester- bzw. Wachsester-Gemische, wie sie z.B. im Jojobaöl oder im Spermöl vorliegen.

Geeignete Dicarbonsäureester (II) sind z.B. Di-n-butyl-adipat, Di-n-butyl-sebacat, Di-(2-ethylhexyl)-adipat, Di-(2-hexyldecyl)-succinat und Di-isotridecyl-acelaat. Geeignete Diolester (III) sind z.B. Ethylenglykoldioleat, Ethylenglykol-di-isotridecanoat, Propylenglykol-di-(2-ethylhexanoat), Propylenglykol-di-isostearat, Propylenglykol-di-pelargonat, Butandiol-di-isostearat und Neopentylglykol-di-caprylat.

Weiterhin eignen sich als Ölkörper verzweigte primäre Alkohole wie sie unter der Bezeichnung Guerbetalkohole (vergl. z.B. A.J.O'Lenick Jr., R.E.Bilbo, Soap Cosm. Chem. Spec. 1987, 52) bekannt sind, z.B. 2-Hexyldecanol oder 2-Octyldodecanol, sowie Ester von Guerbetalkoholen mit langkettigen aliphatischen Carbonsäuren, z.B. mit Stearinsäure.

Die Ölkörper werden in den erfindungsgemäßen O/W-Emulsionen in einer Menge von 5 bis 30 Gew.-%, insbesondere von 10 bis 20 Gew.-%, eingesetzt.

Als Alkylglucoside (B) eignen sich Glucoside mit einem $C_8$- bis $C_{22}$-Alkylrest. Sie werden üblicherweise durch Umsetzung von Glucose oder Stärke bzw. Stärkesirup mit den entsprechenden langkettigen Alkoholen hergestellt. Die Alkohole müssen dabei im Überschuß eingesetzt werden. Nach beendeter Reaktion wird der nicht umgesetzte Alkohol weitgehend abdestilliert, so daß das erfindungsgemäß verwendete Alkylglucosid in der Regel Restalkoholgehalte von ca. 1 Gew.-% enthält.

Alkylglucoside werden durch die allgemeine Formel $RO(G)_x$ charakterisiert, worin bedeuten:
- R einen linearen, gesättigten Alkylrest mit 8 bis 22 C-Atomen
- G eine Glucoseeinheit
- x eine beliebige Zahl zwischen 1 und 10

Die Zahl x wird als Oligomerisationsgrad bezeichnet und stellt einen Mittelwert für die Verteilung von Mono- und Oligoglucosiden dar. Als rechnerisch ermittelte Größe macht der Oligomerisationsgrad eine Aussage über die Verteilung der in einem technischen Oligomerengemisch vorhandenen einzelnen chemischen Individuen, die sich jeweils in der Anzahl der vorhandenen Glucoseeinheiten pro Molekül Alkylglucosid unterscheiden. Bevorzugt soll der mittlere Oligomerisationsgrad x ein Wert von 1,1 bis 1,6 sein.

Eine bevorzugte Ausführungsform der vorliegenden Erfindung ist dadurch gekennzeichnet, daß der Alkylrest des Alkylglucosids eine Kettenlänge von 12 bis 18, insbesondere 16 bis 18, C-Atomen hat. Dabei sind diejenigen Alkylglucoside besonders geeignet, deren $C_{16}$-Anteil mindestens 85 Gew.-% beträgt. Die erfindungsgemäß verwendeten Alkylglucoside sind Spezies, deren Alkylreste die angegebenen bevorzugten Kettenlängen aufweisen; sie können jedoch darüber hinaus noch geringe Anteile an Spezies mit kürzer- und/oder längerkettigen Alkylresten enthalten, wie sie z.B. in den Alkylresten von Fettalkoholen pflanzlichen und tierischen Ursprungs vorliegen.

Es ist besonders bevorzugt, Alkylglucoside mit einem Oligomerisationsgrad x im Bereich von 1,1 bis 1,3 zu verwenden.

Eine weitere bevorzugte Ausführungsform der vorliegenden Erfindung ist dadurch gekennzeichnet, daß sie neben den oben beschriebenen Komponenten zusätzlich einen Konsistenzregler enthält. Unter Konsistenzregler sind dabei Substanzen zu verstehen, die die Konsistenz kosmetischer Präparate, z.B. von Emulsionen, beeinflussen, bestimmen oder erhalten (vergl. H.Fey, I.Otte, "Wörterbuch der Kosmetik", Stuttgart 1985). Als Konsistenzregler können in den erfindungsgemäßen O/W-Emulsionen z.B. langkettige Alkohole, Carboxymethylzellulose oder Salze von Polyacrylsäuren eingesetzt werden. Besonders gut geeignet sind als Konsistenzregler Alkohole mit 14 bis 22, insbesondere mit 16 bis 18 C-Atomen. Der Konsistenzregler kann vorteilhaft in Form des Alkohols eingebracht werden, der nach partieller Abdestillation des nicht umgesetzten Alkohols im Alkylglucosid enthalten ist.

Die thermische Beanspruchung der Alkylglucoside im Zuge der Abdestillation des nicht umgesetzten Alkohols kann leicht zu einer unerwünschten bräunlichen Verfärbung führen, die einen Bleichprozeß erforderlich macht. Es kann daher von Vorteil sein, auf eine zu weitgehende Abtrennung des nicht umgesetzten Alkohols zu verzichten, insbesondere dann, wenn der langkettige Alkohol ohnehin als Konsistenzgeber in der O/W-Emulsion eingesetzt werden soll. Eine weitere Ausführungsform der vorliegenden Erfindung besteht daher darin, daß ein rohes Alkylglucosid eingesetzt wird, dessen Gehalt an nicht umgesetztem Alkohol 1 bis 20 Gew.-%, vorzugsweise 2,5 bis 5 Gew.-%, beträgt.

Die Alkylglucoside werden in den erfindungsgemäßen O/W-Emulsionen in einer Menge von 2 bis 15 Gew.-%, insbesondere von 2 bis 7 Gew.-%, eingesetzt.

Unter Fettsäurepartialglyceriden (C) von gesättigten oder ungesättigten Fettsäuren mit 10 bis 20 C-Atomen sind technische Gemische von Fettsäuremono-, di- und triglyceriden zu verstehen, die durch Veresterung von 1 Mol Glycerin mit 1 bis 2 Mol einer ($C_{10-20}$)-Fettsäure oder durch Umesterung von 1 Mol eines ($C_{10-20}$)-Fettsäuretriglycerids, z.B. von Rindertalg, Schweineschmalz, Palmöl, Sonnenblumenöl oder Sojaöl mit 0,5 bis 2 Mol Glycerin erhalten werden.

Handelsüblich sind zwei Typen von Partialglyceriden. Partialglyceride des Typs I enthalten 35 bis 60 % Monoglyceride, 35 bis 50 % Diglyceride und 1 bis 20 % Triglyceride. Partialglyceride des Typs II werden durch Molekulardestillation aus solchen des Typs I hergestellt und enthalten 90 bis 95 % Monoglyceride, 1 bis 5 % Diglyceride und weniger als 1 % Triglyceride (vergl. dazu: a) G.Schuster und W. Adams: Zeitschrift für Lebensmitteltechnologie, 1979, Band 30(6), S. 256-264; b) G.Schuster (Hrsg.) "Emulgatoren für Lebensmittel", Springer-Verlag, 1985).

Die erfindungsgemäß verwendeten Fettsäurepartialglyceride sollen 60 bis 95 % Monoglyceride, 1 bis 35 % Diglyceride und 0,1 bis 5 % Triglyceride enthalten. In einer bevorzugten Ausführungsform der vorliegenden Erfindung werden Fettsäurepartialglyceride mit einem Monoanteil von 90 bis 95 Gew.-% eingesetzt. Besonders günstig sind Fettsäurepartialglyceride, deren Fettsäurereste Kettenlängen im Bereich von 16 bis 18 C-Atomen haben.

Die Fettsäurepartialglyceride werden in den erfindungsgemäßen O/W-Emulsionen in einer Menge Von 2 bis 20 Gew.-%, insbesondere 4 bis 10 Gew.-%, eingesetzt.

Die erfindungsgemäßen O/W-Emulsionen können darüber hinaus zusätzliche Komponenten bzw. Hilfsstoffe enthalten, die aus dem Stand der Technik bekannt sind. Die wichtigsten sind:

a) Coemulgatoren, z.B. anionaktive Tenside mit Carboxylat-, Sulfonat-, Sulfat- oder Phosphatgruppen wie Seifen, Alkyl- und Arylethersulfate, Fettamine, quartäre Ammonium- und Pyridiniumverbindungen, nichtionische Emulgatoren wie Ethylenoxidaddukte an Alkohole, Carbonsäuren, Partialglyceride und Sorbitanester, amphotere Emulgatoren wie Imidazolinderivate, Betaine oder Sulfobetaine sowie z.B. Fettsäureester und Sorbitanfettsäureester (vergl. z.B. H. Umbach [Hrsg.], "Kosmetik - Entwicklung, Herstellung und Anwendung kosmetischer Mittel", S.86-87, Stuttgart 1988).

b) Feuchthaltemittel, z.B. Glycerin, Polyglycerine, Sorbit, 1,2-Propandiol, 1,2,3-Butan-triol, Polyethylenglykole, Glucose, Mannit, Xylit

c) antimikrobiell wirksame Stoffe als Konservierungsmittel, z.B. Benzoesäure, Salicylsäure, Sorbinsäure, sowie deren Ester und Salze.

d) Parfümöle, z.B. natürliche Riechstoffe, die durch Destillation, Extraktion oder Pressung aus Pflanzen gewonnen werden sowie synthetisch hergestellte Riechstoffe (vergl. z.B. H.Aebi,E.Baumgartner,H.P.Fiedler,G.Ohloff, "Kosmetika, Riechstoffe und Lebensmittelzusatzstoffe", Stuttgart 1978)

e) Antioxidantien, z.B. Tocopherole, Lecithin, Guajakol, Butylkresol, 4-Methyl-2,6-di-tert.-butyl-phenol (BHT), 4-Methoxy-2(3)tert.-butyl-phenol (BHA)

f) Farbstoffe, wie sie z.B. von der Farbstoff-Kommission der Deutschen Forschungsgemeinschaft für Kosmetika zusammengestellt sind ("Färbemittel für Kosmetika" Mitteilung 3, Wiesbaden 1968)

Zur Herstellung der Emulsionen wurden die Alkylglucoside entweder in der heißen Wasser- oder Fettphase gelöst. Vorzugsweise wurde die Wasserphase unter Rühren bei 60 bis 80 °C zu der Ölphase gegeben. Die so erhaltenen Dispersionen wurden anschließend auf 30 °C abgekühlt. Gewünschtenfalls kann durch Geräte, die nach dem Rotor-Stator-Prinzip arbeiten, z.B. Ultra Turrax T50 (Ika-Werke), die Struktur der Emulsion während des Abkühlvorgangs noch weiter verbessert werden.

Die Herstellung der erfindungsgemäßen O/W-Emulsionen kann aufgrund der Wasserlöslichkeit der Alkylglucoside, insbesondere solcher mit Alkylresten von 12 bis 16 C-Atomen, auch auf kaltem Wege erfolgen. Insbesondere in Kombination mit hydrophilen oder hydrophoben Emulgatoren und Ölen, die bei Raumtemperatur flüssig sind, können unter Verwendung polymerer Verdickungsmittel auf diese Weise gehaltvolle flüssige oder cremeförmige kosmetische Emulsionen formuliert werden.

Ein weiterer Gegenstand der Erfindung sind Emulgatorkonzentrate zur Herstellung der erfindungsgemäßen O/W-Emulsionen enthaltend

(D) 24 bis 75 Gew.-% ein oder mehrerer Alkylglucoside der allgemeinen Formel $RO(G)_x$, worin bedeuten
- R einen linearen, gesättigten Alkylrest mit 8 bis 22 C-Atomen
- G eine Glucoseeinheit
- x eine beliebige Zahl zwischen 1 und 10

(E) 24 bis 75 Gew.-% eines Fettsäurepartialglycerids mit einem Monoanteil von 60 bis 95 Gew.-%

5

(F) 0,1 bis 50 Gew.-% eines linearen, gesättigten Alkohols mit 14 bis 22, insbesondere mit 16 bis 18 C-Atomen.

Aus den oben genannten Gründen können zur Herstellung derartiger Emulgatorkonzentrate auch Alkylglucoside eingesetzt werden, bei denen der im Zuge der Herstellung nicht umgesetzte Alkohol entweder gar nicht oder nur partiell abdestilliert wurde. Eine bevorzugte Ausführungsform der vorliegenden Erfindung besteht daher darin, daß die Emulgatorkonzentrate unter Verwendung von Alkylglucosiden hergestellt werden, deren Gehalt an nicht umgesetztem Alkohol 3 bis 90 Gew.-%, bevorzugt 3 bis 40 Gew.-%, beträgt. In derartigen Fällen kann die Abmischung der Alkylglucoside mit den Fettsäurepartialglyceriden unmittelbar nach Beendigung der Reaktion zwischen Glucose und langkettigen Alkoholen und gewünschtenfalls partieller Abdestillation nicht umgesetzten Alkohols bei Temperaturen von etwa 100 °C erfolgen. Auf diese Weise lassen sich selbstemulgierende Emulsionsgrundlagen herstellen, mit denen sich kosmetisch anspruchsvolle Cremes bzw. Lotionen formulieren lassen.

Die erfindungsgemäßen Emulgatorkonzentrate können darüber hinaus zusätzliche Komponenten bzw. Hilfsstoffe enthalten, wie sie oben beschrieben sind, z.B. Ölkörper, Coemulgatoren, Feuchthaltemittel, Konservierungsmittel, Parfümöle, Antioxidatien, Farbstoffe.

Die folgenden Beispiele sollen den Erfindungsgegenstand näher erläutern.

**Beispiele**

1. Verwendete Substanzen
    1.1. Ölkörper (A)
    Cetiol V: Ölsäuredecylester (Fa. Henkel KGaA/Düsseldorf); Myritol 318: Capryl-/Caprinsäuretriglycerid (Fa. Henkel KGaA/Düsseldorf)
    1.2. Alkylglucoside (B)
    Als Alkylglucoside wurden $C_{14}$-, $C_{16}$- und $C_{18}$-Alkylglucoside (abgekürzt als AG C14, AG C16 und AG C18) mit Oligomerisationsgraden von x = 1,2 bis 1,4 eingesetzt. Zur Herstellung der $C_{14}$-, $C_{16}$- und $C_{18}$-Alkylglucoside wurden verwendet: Glucose und die jeweiligen $C_{14}$-, $C_{16}$- bzw. $C_{18}$-Fettalkohole (C-Kettenverteilung nach GC: a) für $C_{14}$-Fettalkohol: 2% $C_{12}$; 96% $C_{14}$; 2% $C_{16}$; b) für $C_{16}$-Fettalkohol: 2% $C_{14}$; 96% $C_{16}$; 2% $C_{18}$; c) für $C_{18}$-Fettalkohol: 2% $C_{16}$; 96% $C_{18}$; 2% $C_{20}$).
    1.3. Fettsäurepartialglyceride (C)
    Cutina MD: Fettsäurepartialglycerid auf Basis von gehärtetem Palmöl mit einem Monoanteil von 42 Gew.-% (Fa. Grünau/Illertissen); Monomuls 60-35: Fettsäurepartialglycerid auf Basis von gehärtetem Palmöl mit einem Monoanteil von 60 Gew.-% (Fa. Grünau/Illertissen); Monomuls 90-35: Fettsäurepartialglycerid auf Basis von gehhärtetem Palmöl mit einem Monoanteil von 90 Gew.-% (Fa. Grünau/Illertissen)
    1.4. Weitere Komponenten
    Lanette O: Cetyl-/Stearylalkohol [50:50] (Fa. Henkel KGaA/Düsseldorf); Lanette E: Cetyl-/Stearylalkoholsulfat,Na-Salz [50:50] (Henkel KGaA/Düsseldorf); Cutina E-24:Polyoxyethylen24-Glycerinmonostearat (Fa. Henkel KGaA/Düsseldorf).
2. Herstellung und Charakterisierung der Dispersionen
    Zur Herstellung der Emulsionen wurden die Alkylglucoside entweder in der heißen Wasser- oder Fettphase gelöst. Vorzugsweise wurde die Wasserphase unter Rühren bei 60 bis 80 °C zu der Ölphase gegeben. Die so erhaltenen Dispersionen wurden anschließend auf 30 °C abgekühlt.
    Nach 24 Stunden wurden die Viskositäten bei 23 °C mit einem Brookfield-Viskosimeter unter Verwendung von Spindel E bei 4 UpM gemessen. Die Emulsionen wurden anschließend zwei Wochen jeweils bei -5 °C, 20 °C sowie 40 °C gelagert und visuell sowie bei 400-facher Vergrößerung unter dem Mikroskop beurteilt. Die Ergebnisse sind in Abhängigkeit von der Zusammensetzung der Emulsionen in Tabelle 1 zusammengestellt.
    In allen Fällen, bei denen als Partialglyceride Monomuls 90-35 bzw. Monomuls 60-35 (Monoglyceridgehalt: 90% bzw. 60%) verwendet wurden, wurden Emulsionen erhalten, die sowohl unmittelbar nach ihrer Herstellung, als auch nach der oben beschriebenen Lagerung homogen waren (Tabelle 1, Spalten B1 bis B8). Im Vergleich dazu lag bei Verwendung von Cutina MD, einem Partialglycerids mit einem Monoglyceridgehalt von 42%, entweder direkt nach der Herstellung eine zwar eine homogene Emulsion vor, die jedoch bei 400-facher Vergrößerung unter dem Lichtmikroskop deutlich kristalline Bestandteile zeigte und sich bei 40 °C in zwei Phasen trennte (Tabelle 1, Spalten V1 und V2) oder die Emulsion war bereits unmittelbar nach der Herstellung makroskopisch inhomogen (Tabelle 1, Spalte V3).

Tabelle 1: O/W-Emulsionen

| | V1 | V2 | V3 | B1 | B2 | B3 | B4 | B5 | B6 | B7 | B8 |
|---|---|---|---|---|---|---|---|---|---|---|---|
| AG C14 (x=1,4) | – | 4,0 | – | – | – | – | – | – | 4,0 | – | – |
| AG C16 (x=1,2) | 4,0 | – | – | 4,0 | – | – | – | – | – | – | – |
| AG C16 (x=1,3) | – | – | 4,0 | – | 4,0[1] | 4,0 | 4,0 | 4,0 | – | 4,0 | 4,0[5] |
| AG C18 (x=1,4) | – | – | – | – | – | – | – | – | – | – | – |
| Monomuls 90-35 | – | – | 7,0 | – | – | – | 7,0 | 7,0 | – | 7,0 | 4,0[5] |
| Monomuls 60-35 | 5,6 | 5,6 | – | 5,6 | 5,0[1] | 5,6 | – | – | 5,6 | – | – |
| Cutina MD | 14,0 | 14,0 | 12,0 | 14,0 | 14,0 | 14,0 | 12,0 | 12,0 | 14,0 | 12,0 | 14,0 |
| Cetiol V | – | – | – | – | – | – | – | – | – | – | – |
| Myritol 318 | – | – | – | – | – | – | – | – | – | – | 0,8[5] |
| Stearylalkohol | – | – | – | – | 1,0[1] | – | – | – | – | – | 1,0 |
| Cetylalkohol | 0,8 | 0,8 | – | 0,8 | – | 0,8 | – | – | 0,8 | – | – |
| Lanette O | 3,0 | 3,0 | 3,0 | 3,0 | 3,0 | 5,0 | 5,0 | 5,0 | 3,0 | 3,0 | 3,0 |
| Glycerin 86% | – | – | – | – | – | – | 0,5 | 1,0 | – | 3,0 | – |
| Lanette E | – | – | – | – | – | – | – | – | – | – | – |
| Cutina E-24 | – | – | – | – | – | – | – | – | – | – | – |
| Wasser, Konservierung | ad 100 | ad 100 | ad 100 | ad 100 | ad 100 | ad 100 | ad 100 | ad 100 | ad 100 | ad 100 | ad 100 |
| Viskosität [Pas] | 180 | 190 | 75 | 100 | 140 | 150 | 250 | 250 | 150 | 100 | 100 |
| **Aussehen nach Lagerung bei:** | | | | | | | | | | | |
| -5 °C | hom[2] | hom | inhom[4] | hom | hom | hom | hom | hom | hom | hom | hom |
| 20 °C | hom | hom | inhom | hom | hom | hom | hom | hom | hom | hom | hom |
| 40 °C | Trenn[3] | Trenn | Trenn | hom | hom | hom | hom | hom | hom | hom | hom |

1) Beispiel B2 wurde aus einem Emulgatorkonzentrat, bestehend aus einer 1:1 Mischung von AG C16 (Restalkoholgehalt: 20%) und Monomuls 60-35, hergestellt.
2) hom = homogen
3) Trenn = Trennung
4) inhom = inhomogen
5) Beispiel B8 wurde aus einem Emulgatorkonzentrat, bestehend aus einer Mischung von 40% AG C16 (Restalkoholgehalt: 20%), 52% Monomuls 90-35 und 8% Stearylalkohol, hergestellt.

## Patentansprüche

1. Öl-in-Wasser-Emulsionen mit einem Gehalt an Alkylglucosiden, dadurch gekennzeichnet, daß sie enthalten

(A) einen oder mehrere wasserunlösliche Ölkörper,

(B) als Emulgator ein oder mehrere Alkylglucoside der allgemeinen Formel RO(G)$_x$, worin bedeuten
- R einen linearen, gesättigten Alkylrest mit 8 bis 22 C-Atomen
- G eine Glucoseeinheit
- x eine beliebige Zahl zwischen 1 und 10,

(C) als Kristallisationsinhibitor ein Fettsäurepartialglycerid mit einem Monoanteil von 60 bis 95 Gew.-%.

2. Emulsionen nach Anspruch 1, dadurch gekennzeichnet, daß der wasserunlösliche Ölkörper (A) in einer Menge von 5 bis 30 Gew.-%, das Alkylglucosid (B) in einer Menge von 2 bis 15 Gew.-% und das Fettsäurepartialglycerid (C) in einer Menge von 2 bis 20 Gew.-% enthalten ist.

3. Emulsionen nach Anspruch 1 oder 2, dadurch gekennzeichnet, daß der Alkylrest des Alkylglucosids (B) eine Kettenlänge von 16 bis 18 C-Atomen hat.

4. Emulsionen nach mindestens einem der Ansprüche 1 bis 3, dadurch gekennzeichnet, daß der Oligomerisationsgrad x des Alkylglucosids (B) 1,1 bis 1,6 beträgt.

5. Emulsionen nach mindestens einem der Ansprüche 1 bis 4, dadurch gekennzeichnet, daß zusätzlich ein Alkohol mit 14 bis 22 C-Atomen, insbesondere mit 16 bis 18 C-Atomen, als Konsistenzregler enthalten ist.

6. Emulgatorkonzentrate zur Herstellung lagerstabiler Öl-in-Wasser-Emulsionen nach mindestens einem der Ansprüche 1 bis 5, dadurch gekennzeichnet, daß sie enthalten

(D) 24 bis 75 Gew.-% ein oder mehrerer Alkylglucoside der allgemeinen Formel RO(G)$_x$, worin bedeuten
- R einen Alkylrest mit 8 bis 22 C-Atomen
- G eine Glucoseeinheit
- x eine beliebige Zahl zwischen 1 und 10

(E) 24 bis 75 Gew.-% eines Fettsäurepartialglycerids mit einem Monoanteil von 60 bis 95 Gew.-%

(F) 0,1 bis 50 Gew.-% eines linearen, gesättigten Alkohols mit 14 bis 22, insbesondere mit 16 bis 18 C-Atomen.

**Claims**

1. Oil-in-water emulsions containing alkyl glucosides, characterized in that they contain

(A) one or more water-insoluble oils,

(B) as emulsifier, one or more alkyl glucosides corresponding to the general formula RO(G)$_x$, in which
- R is a linear saturated $C_{8-22}$ alkyl radical
- G is a glucose unit
- is a number in the range from 1 to 10,

characterized in that they contain

(C) a fatty acid partial glyceride with a monoglyceride content of 60 to 95% by weight as crystallization inhibitor.

2. Emulsions as claimed in claim 1, characterized in that the water-insoluble oil (A) is present in a quantity of 5 to 30% by weight, the alkyl glucoside (B) is present in a quantity of 2 to 15% by weight and the fatty acid partial glyceride (C) is present in a quantity of 2 to 20% by weight.

3. Emulsions as claimed in claim 1 or 2, characterized in that the alkyl radical of the alkyl glucoside (B) has a chain length of 16 to 18 carbon atoms.

4. Emulsions as claimed in at least one of claims 1 to 3, characterized in that the degree of oligomerization x of the alkyl glucoside (B) is 1.1 to 1.6.

5. Emulsions as claimed in at least one of claims 1 to 4, characterized in that they additionally contain a $C_{14-22}$ and, more particularly, $C_{16-18}$ alcohol as consistency regulator.

**6.** Emulsifier concentrates for the production of the storable oil-in-water emulsions claimed in at least one of claims 1 to 5, characterized in that they contain

(D) 24 to 75% by weight of one or more alkyl glucosides corresponding to the general formula RO-$(G)_x$, in which

- R is a linear saturated $C_{8-22}$ alkyl radical
- G is a glucose unit
- x is a number in the range from 1 to 10,

(E) 24 to 75% by weight of a fatty acid partial glyceride containing 60 to 95% by weight monoglyceride

(F) 0.1 to 50% by weight of a linear saturated alcohol containing 14 to 22 carbon atoms and, more particularly, 16 to 18 carbon atoms.


**Revendications**

**1.** Emulsions d'huile dans l'eau avec un contenu en alkylglucosides, caractérisées en ce qu'elles contiennent :

(A) une ou plusieurs substances de l'huile

(B) comme émulsionnant un ou plusieurs alkylglucoside de formule générale RO$(G)_x$, dans laquelle,

- R représente un radical alkyle linéaire, saturé avec 8 à 22 atomes de C,
- G une unité glucose
- x un noire guelconque entre 1 et 10,

(C) code inhibiteur de cristallisation un glycéride partiel d'acide gras avec une proportion de monoglycéride de 60 à 95 % en poids.

**2.** Emulsions selon la revendication 1, caractérisées en ce que la substance de l'huile (A) soluble dans l'eau est contenue en une quantité de 5 à 30 % en poids, l'alkylglucoside (B) en une quantité de 2 à 15 % en poids et le glycéride partiel d'acide gras (C) en une quantité de 2 à 20 % en poids.

**3.** Emulsions selon les revendications 1 ou 2, caractérisées en ce que le radical alkyle de l'alkylglucoside (B) a une longueur de chaîne de 16 à 18 atomes de C.

**4.** Emulsions selon au moins une des revendications 1 à 3, caractérisées en ce que le degré d'oligomérisation x de l'alkylglucoside (B) va de 1,1 à 1,6.

**5.** Emulsions selon au moins une des revendications 1 à 4, caractérisées en ce qu'en supplément est contenu conme régulateur de consistance un alcool avec 14 à 22 atomes de C, en particulier avec 16 à 18 atomes de C.

**6.** Concentrés d'émulsionnant pour produire des émulsions d'huile dans l'eau stables au stockage selon une au moins des revendications 1 à 5, caractérisés en ce qu'ils contiennent :

D) 24 à 75 % en poids d'un ou de plusieurs alkylglucosides de formule générale RO$(G)_x$, dans laquelle :

- R est un radical alkyle linéaire, saturé avec de 8 à 22 atomes de C
- G une unité glucose
- x un nombre quelconque entre 1 et 10.

E) 24 à 75 % en poids d'un glycéride partiel d'acide gras avec une proportion de monoglycéride de 60 à 95 % en poids.

F) 0,1 à 50 % en poids d'un alcool linéaire, saturé avec de 14 à 22, en particulier de 16 à 18 atomes de C.